# EUROPEAN PATENT APPLICATION

(11) **EP 4 512 737 A1**
(43) Date of publication of application: **26.02.2025**
(21) Application number: 23193252.6
(22) Date of filing: 24.08.2023
(51) Int. Cl.: B65D 75/00, A61M 3/02

(54) **STANDING BAG FOR IRRIGATION APPLICATION**

(71) Applicant: B. Braun Melsungen AG, 34212 Melsungen (DE)
(72) Inventor: MÉDICO, Léonard, 1895 Vionnaz (CH)
(74) Representative: Maiwald GmbH

(57) **Abstract**

A bag suitable for being used in an upright position in an irrigation application, the bag comprising at least two walls, at least one base member, wherein the at least two walls and the at least one base member are connected forming liquid-tight connections, wherein the at least two walls, the at least one base member, and the liquid-tight connections form a liquid-tight cavity, wherein the at least one base member and the connections between the base member and the at least two walls form a base part, and wherein the at least two walls and the connection between the at least two walls form an upper part.

## Description

### Technical Field of the Invention

The present invention is located in the field of containers for irrigation application, the use of such containers in irrigation application, and a method for irrigating an object during surgery with such a container.

### Background of the Invention

Irrigation containers are usually used to drain and wash surgery fields, such as body cavities, tissues, wounds, and burns, during surgeries. Typically, irrigation containers are bottles comprising a sterile liquid, wherein the bottle should be openable by hand. Usually, during operation, the sterile liquid is poured out thereby ensuring that the sterile status of the liquid is kept intact up until the liquid contacts the surgery field. Nowadays, normally blown bottles made from plastic material are used to achieve this simple functionality.

Current technologies, such as injection blow molding, injection stretch blow molding, extrusion blow molding, and compression blow molding, do not allow for producing such blown bottles having walls with a thickness of below 200 µm. Additionally, bottom and top parts of such blown bottles are usually even thicker than their walls and may present weld lines (e.g., when produced by extrusion blow molding), which are locally thicker and can be the source of weak points).

However, the thickness of the walls of such blown bottles defines their overall weight. High weight has the disadvantage of higher transportation costs and generally more demanding handling,.

Furthermore, the production of such blown bottles affords, due to the thickness of their walls, a high plastic consumption. Moreover, during extrusion blow molding of such blown bottles, a lot of scrap material results from both the neck and bottom areas, which are cut from the parison to shape the container. Thus, another disadvantage of such blown bottles is their high plastic consumption and plastic waste creation during production of the bottles.

Sustainability is getting more and more in focus of the packaging industry. Likewise, the pharma customers are asking for sustainable characteristics of provided containers. Hence, more sustainable packaging provides market advantage and potential cost saving together with positive impact on the carbon footprint.

To solve this problem, GB 1287668 A discloses a bag for irrigation solution. However, this bag cannot be used at all in standing mode. Thus, the bags of GB 1287668 A cannot stand in upright position. Hence, they can only rest either flat on their side or in a hanging position. Thus, they are used in a hanging position during infusion application, unlike blown bottles, which can be rested in upright position during irrigation application.

EP 2 767 270 A1 and WO 2010/137338 A1 suggest bags made from plastic material, which are capable of being stored in an upright position. However, they are made for infusion application. Hence, they need to be connected to an IV set and hung by a hanger when being used to provide the infusion solution to the patient.

Stand up pouches are known from use for enteral nutrition. Such pouches, however, do not require terminal sterilization and are not subjected to the same biocompatibility, pharmacopeias, and overall technical requirements as irrigation products. Furthermore, stand up pouches are used within the food, pet care, or technical fluids industry. However, their content eventually requires only pasteurization, and thereby heating up to only 70 to 90 °C, but no sterilization.

### Summary of the Invention

Hence, there is the need for containers for irrigation application having reduced weight and reduced plastic consumption during production.

Thus, it is an object of the present invention to provide a container for irrigation application, which has reduced wall thickness and can be used in upright position.

It has now been surprisingly found out that above-mentioned object can be achieved by a bag suitable for being used in an upright position in an irrigation application, the bag comprising at least two walls, and at least one base member, wherein the at least two walls and the at least one base member are connected forming liquid-tight connections, wherein the at least two walls, the at least one base member, and the liquid-tight connections form a liquid-tight cavity, wherein the at least one base member and the connections between the base member and the at least two walls form a base part, and wherein the at least two walls and the connection between the at least two walls form an upper part.

It has been further found that above-mentioned object can be achieved by the use of said bag in an irrigation application.

Finally, it has been further found out that above-mentioned object can be achieved by a process for irrigating an object, providing the following steps: providing the object; providing a bag suitable for being used in an upright position in an irrigation application, the bag comprising at least two walls, at least one base member, optionally at least one gripping means, and optionally at least one outlet, wherein the at least two walls and the at least one base member are connected forming liquid-tight connections, wherein the at least two walls, the at least one base member, and the liquid-tight connections form a liquid-tight cavity, wherein the at least one base member and the connections between the base member and the at least two walls form a base part, and wherein the at least two walls and the connection between the at least two walls form an upper part; opening the bag; and holding the bag with one hand and applying the liquid held in the bag to the object by pouring.

The advantage of the present invention is that the standing bag can be used in standing operation during irrigation application. In comparison to blown bottles typically used in irrigation application, the present invention has the advantage of less material usage, less cut off material during production and generally higher sustainability due to lower waste weight. As the hanging operation is avoided, the need for special equipment such as a stand, tubes, etc. is removed. Furthermore, operation is accelerated as a change of the bag in case the sterile liquid has been consumed, can be achieved much faster without the need of the extra equipment.

### Brief Description of the Drawings

- Figure 1: shows a schematic drawing of the shape of the standing bag of the most general embodiment of the present invention.
- Figure 2: shows a schematic drawing of the shape of the standing bag of a preferred embodiment of the present invention comprising corners.
- Figure 3: shows a schematic drawing of the shape of a standing bag of a preferred embodiment of the present invention comprising a top member.

### Definitions

The term "*irrigation solution*" as used herein denotes a liquid used in irrigation application. Irrigation solutions have to be sterile. An irrigating solution is suitable for killing microorganisms, disrupting the biofilm on surgery objects, inactivating virulence factors such as endotoxin, dissolving pulp-tissue remnants, removing hard-tissue debris and the smear layer created during instrumentation or prevent their formation, providing lubrication for instruments, and being biocompatible. Usually, an irrigation solution is based on water. Optionally, it can be mixed with an adjuvant. Adjuvants may be sodium chloride, sodium hypochlorite, potassium chloride, calcium chloride, chlorhexidine, ethylenediamine tetraacetic acid, citric acid, etidronic acid, maleic acid, sodium lactate, or mixtures thereof. Most preferably, irrigation solutions are selected from the list consisting of 0.9% aqueous sodium chloride solution, Ringer's lactate solution, and distilled water.

The term *"surgery object"* as used herein denotes an object, preferably body part, which can be treated by surgery, wherein the body part is preferably selected from a group consisting of body cavities, tissues, wounds, bones, and burns.

The term *"irrigation"* or *"irrigation application"* as used herein denotes the process of mechanical cleansing of surgery objects during topical, intra- or postoperative surgical interventions using an irrigation solution. This is typically achieved by pouring the irrigation solution on the surgery object and rinsing it. Alternatively, such a process is achieved by pouring out liquid in an intermediate recipient (like a kidney bowl), from which the solution is then taken out with a syringe to be spread onto the object. The goal of the irrigation is to clear a surgical object from, e.g., blood and tissue to keep the surgery object sterile, prevent infections, moisten the surgery object, wound tamponades, cloths, bandages, and dressings, cleansing of operating instruments and accessories, and/or make the object visible for the surgeon. It is understood that prior to and/or during the process of irrigation, the container comprising the irrigation solution has to be put aside, at least one time.

The term *"upright position"* as used herein denotes an orientation of the bag of the present invention in that each distance of each point in the base part of the bag to the center of the gravitational force is smaller than in any point of the upper part and optionally the top part of the bag.

The term *"used in an upright position"* as used herein in particular in connection with the irrigation application denotes an irrigation process, in which the container is used such that the container is in upright position when being put aside.

The term *"sterile"* as used denotes the status of an object having a significantly reduced number of bacteria and/or viruses on its surface to reduce the risk of an infection. In particular, the term *"sterile"* denotes an object or substance, which has a bioburden load of lower than 10⁻⁶. The bioburden load can be measured i.e., according to ISO 11737-1:2018.

The term *"sterilization"* as used herein denotes a method to destroy all forms of living microorganisms from a substance. As there is always a certain probability of at least one microorganism to survive such procedure, the aim of sterilization is the reduction of initially present microorganisms or other potential pathogens. Generally, sterilization is accepted to be achieved if the bioburden load of the substance of object to be sterilized is lower than 10⁻⁶. The bioburden load can be measured i.e., according to ISO 11737-1:2018. Sterilization can be achieved using several methods. In one sterilization process the object is heated up to at least 105 °C to achieve a sterile object. Thereby, the object should not be deformed by the elevated temperature. Preferably, the heating step is performed in an autoclave. In another sterilization process, the object is brought into contact with toxic gases such as a mixture of ethylene oxide and carbon dioxide. Filtration methods are also used to sterilize liquids, i.e., by using membrane filters, Seitz filters, and/or candle filters. Finally, sterilization can be achieved by indirect energy import into or onto the object, e.g., by ultrasonic waves, ultraviolet light, as well as by high energy particles (such as electrons, gamma- or X-rays).

The term *"liquid-tight"* as used herein denotes a quality of an object to function as a barrier for a liquid, preferably for a water-based liquid.

The term *"seam"* as used herein denotes an area of at least two connected walls including at least one edge of each wall, of a bag, at which the two walls are connected, i.e., by gluing or welding, thereby forming a seam area, a seam edge, and an inner boundary of the edge.

The term *"seam width"* as used herein denotes the distance between the seam edge and the inner boundary of the seam in a direction perpendicular to the seam edge. Usually, the seam width is substantially homogeneous, i.e., does not vary in the direction of the seam edge. However, variation of the seam width can be part of the overall design of the bag.

The term *"seam thickness"* relates to the thickness of the combined thicknesses of the walls connected in the seam.

### Detailed Description of the Invention

In the following, the present invention will be described in detail. Thus, the apparatus, i.e., the bag, the use thereof and a method using said bag are described in the following.

### Bag of the present invention

The present invention relates to a bag suitable for being used in an upright position in an irrigation application, the bag **(1)** comprising:
- at least two walls **(2),** and
- at least one base member **(3),**

wherein the at least two walls **(2)** and the at least one base member **(3)** are connected forming liquid-tight connections **(6),**
wherein the at least two walls **(2),** the at least one base member **(3),** and the liquid-tight connections **(6)** form a liquid-tight cavity **(7),**
wherein the at least one base member **(3)** and the connections **(6a)** between the base member **(3)** and the at least two walls **(2)** form a base part **(8),** and
wherein the at least two walls **(2)** and the connection **(6b)** between the at least two walls **(2)** form an upper part **(9).**

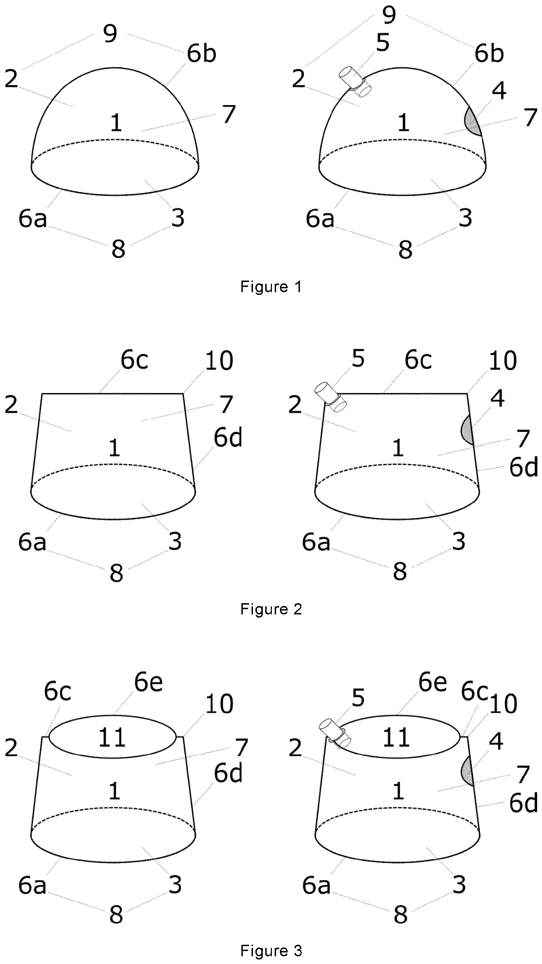

As respective schematic drawing of the shape of such an embodiment of the bag **(1)** of the present invention can be found in Figure 1. Preferably, the bag comprises at least one gripping means **(4)** and/or at least one outlet **(5).** Preferably, the base member **(3)** of the bag **(1)** of the present invention does not comprise an outlet and/or gripping means, preferably does not comprise any outlet and gripping means. This improves the standing capabilities of the bag **(1),** as no means outstand the base member **(3).**

Preferably, the base member is formed as a plane, i.e., a flat area. More preferably, the base member is formed as a plane not comprising any edges. Hence, even more preferably, the base member is formed as an ellipsis. To enhance the static of the bag, the base member preferably provides at least one folding reaching into the liquid-tight cavity **(7).** Typically, standing bags are formed from a single sheet of plastic foil, which is folded twice at the bottom. Usually, the base member is called a "gusset".

The connections **(6)** of the bag of the present invention can be formed by any method allowing for a liquid-tight connection. Preferably, the connections **(6)** are formed by gluing or by welding. Most preferably, the connections **(6)** are formed by welding, i.e., by plastic welding. Usually, the connections are present as a plane, i.e., a flat area, in which the two entities to be connected, i.e., the at least two walls **(2),** constantly are in contact with each other. Depending on the use case, this area can be enlarged, i.e., to attach further equipment, print information thereon, or stance orifices therein. Usually, the connections, which are used for forming the liquid-tight connection and definition of the liquid-tight cavity **(7)** only, should not be too small, as the liquid tight connection depends on the size of the area of contact, but also not too large, as otherwise the size of the bag gets too large without any further volume increase wasting space in the stores. Hence, preferably, the connection **(6)** has a width equal to or more than 1 mm, preferably equal to or more than 5 mm, and most preferably equal to or more than 8 mm. Usually, the connection **(6)** has a width of not more than 10 mm.

For the bag **(1)** of the present invention to facilitate the irrigation solution to leave, it is advantageous if the volume of the cavity **(7)** can be adjusted during operation. Hence, preferably, the base member **(3)** of the bag **(1)** of the present invention is configured to move at least partially into the liquid-tight cavity **(7),** thereby reducing the volume of the liquid-tight cavity **(7).** More preferably, the base member **(3)** is foldable. Most preferably, the base member is foldable so that the fold extends into the liquid-tight cavity **(7).** This allows not only a reduction of the volume of the liquid-tight cavity **(7)** by the fold reaching into the liquid-tight cavity **(7),** but also by the at least two walls moving towards each other. Thereby, the volume of the liquid-tight cavity **(7)** can be significantly reduced up to a point of less than 5% of the initial volume, preferably less than 3%, and most preferably less than 1%. The base member could be rigid. In that case a means to facilitate folding has to be present in the base member, i.e., a hinge. Preferably, however, the base member comprises a flexible material, preferably a polymer material.

Preferably, the bag **(1)** of the present invention comprises at least one outlet **(5).** The at least one outlet **(5)** is used to pour the irrigation solution out of the bag on the surgery object or field. Thus, the at least one outlet **(5)** comprises a means for opening, thereby connecting, the liquid-tight cavity **(7)** with the environment. Preferably, the outlet **(5)** can be irreversibly or reversibly opened by the means for opening . Hence, in one preferred embodiment of the present invention, the at least one outlet **(5)** provides at least one means for irreversibly opening the outlet **(5),** i.e., to tear-off the liquid-tight cavity **(7),** i.e., by tearing off one of the at least two walls **(2).** Such means can be a single slit in one of the connections **(6)** of the at least two walls **(2),** but also a tear-off opening or a peel-off opening. In such an embodiment, the bag can be opened such as known from a milk carton by tearing the edge or any desired part of the bag open.

In another preferred embodiment, the at least one outlet **(5)** provides at least one means for reversibly opening the outlet **(5),** such as a screw cap. Hence, preferably, the at least one outlet **(5)** of the bag of the present invention comprises at least one means for opening the outlet **(5),** which is selected from the list consisting of a tear-off opening, a spout, a break-off opening, a screw-off opening, a twist-off opening, a peel-off opening, a flip-flop cap opening, a valve, and combinations thereof. Preferably, the spout can be selected from the list consisting of a screw cap, a tear-off cap, a squeeze valve, and a spray connector. The at least one outlet **(5)** is preferably rigid, such as a rigid spout. This ensures operation of the at least one outlet **(5)** even if the bag has already collapsed to a significant extend. Most preferably, the at least one outlet **(5)** is integrated into the at least two walls **(2)** or the connections **(6).** Thus, preferably, the at least one outlet **(5)** is glued or welded into the at least two walls **(2)** or the connections **(6).**

In a specifically preferred embodiment, the at least one outlet **(5)** comprises a tube being welded between the two walls **(2),** thereby forming a channel, wherein the outer part of the tube is closed by a welded seam between the two walls **(2),** wherein the seam has a means to tear off the tube, i.e., by tearing off one of the at least two walls **(2).** Such means can be a single slit in one of the connections **(6)** of the at least two walls **(2),** but also a tear-off opening.

Preferably, the at least one outlet **(5)** comprises a tamper-evidence feature. The tamper-evidence feature design depends on the design of the at least one outlet **(5).** Usually, the skilled person knows how to implement a tamper-evidence feature into an outlet.

Preferably, the at least one outlet **(5)** and/or the at least one gripping means **(4)** are comprised in the upper part **(9)** of the bag of the present invention, preferably in the connection **(6b)** between the at least two walls **(2).** The location of the at least one outlet **(5)** and the at least one gripping means **(4)** in the upper part further improves the standability of the bag. Locating the at least one outlet **(5)** and/or the at least one gripping means **(4)** in the connection **(6b)** achieves two advantages. First of all, the connections form part of the bag having best resilience ensuring reduction of material failure during operation. Furthermore, locating the at least one gripping means **(4)** in the connection and not in the at least two walls avoids that the two walls are pressed together during operation and the volume of the liquid-tight cavity **(7)** is decreased thereby affecting the speed and control of the flow of the irrigation liquid out of the bag. Thus, the location of the at least one gripping means **(4)** in the connection **(6)** allows for better control of the speed of the outflow.

Preferably, the distance of the at least one outlet **(5)** to the base member is larger than the distance of the at least one gripping means **(4)** to the base member. Likewise, preferably, the at least one outlet **(5)** and the at least one gripping means **(4)** are located on opposite sides of the liquid-tight cavity **(7).** Particularly, in case the at least one outlet **(5)** and the at least one gripping means **(4)** are located in the connection **(6b),** the at least one gripping means **(4)** is located in first part of the connection **(6b)** extending from the base member **(3)** to a point of the connection **(6b)** having a maximum distance to the base member **(3)** and wherein the at least one outlet **(5)** is located in a second part of the connection **(6b)** extending from the point of the connection **(6b)** having a maximum distance to the base member **(3),** wherein the first part and the second part of the connection **(6b)** are not identical. This has the positive effect of a well-balanced gravity center of the bag during irrigation operation, in particular if the bag is used by one hand only and the irrigation solution is poured on the surgery field or object.

In a first preferred embodiment of the present invention as depicted in Figure 2, in the bag the connection **(6b)** of the at least two walls **(2)** comprises at least two corners **(10).** Such a design facilitates the automatic formation of the connections **(6b),** as the corners allow for a better hold. Furthermore, the corners of the walls reduce the amount of scrap material during production of the bag.

More preferably, each of the at least two walls is a plane having four corners, preferably a rectangle. Such a design allows for improved automatic processing and even more reduce scrap material.

Preferably, in the first preferred embodiment of the present invention, the at least one outlet **(5)** is located in the connection **(6c)** between the at least two walls **(2)** extending between the at least two corners **(10).** This ensures that the distance between the at least one outlet **(5)** and the base member is maximized. It further ensures improved attachment of the at least one outlet **(5)** in the bag. Furthermore, the at least two walls **(2)** form together with the connection **(6c)** a V-shape, which acts as a funnel allowing for easy and complete removal of the irrigation solution through the at least one outlet **(5).** More preferably, the at least one outlet **(5)** is located in one of the at least two corners **(10).** This strengthens the above-mentioned effects, as the at least one outlet **(5)** will be held by the two connections **(6d, 6c)** joining in the corners allowing for even higher stability. Furthermore, the at least two walls **(2)** form together with each corner and connections **(6d, 6c)** a funnel shape, further improving the removal effect of the irrigation solution during pouring.

Also preferably, in the first preferred embodiment of the present invention, the at least one gripping means **(4)** is located in the at least two walls **(2)** or in one of the connections **(6d)** between the at least two walls **(2)** extending between one of the at least two corners **(10)** and the connection **(6a)** between the base member **(3)** and the at least two walls **(2).** Preferably, the at least one gripping means **(4)** is located in one of the connections **(6d)** between the at least two walls **(2)** extending between one of the at least two corners **(9)** and the connection **(6a)** between the base member **(3)** and the at least two walls **(2).** This achieves that the distance of the at least one gripping means **(4)** to the base member **(3)** is smaller than the distance of the at least one outlet **(5)** to the base member **(3)** resulting in an improved balance of the gravitational center of the bag during irrigation application, i.e., pouring out the irrigation solution.

Hence, most preferably, in the first preferred embodiment of the present invention, the at least one gripping means **(4)** is located in one connection **(6d)** between the at least two walls **(2)** extending between one of the at least two corners **(9)** and the at least one outlet **(5)** is located in the connection **(6c)** between the at least two walls **(2)** extending between a different corner of the at least two corners **(10),** preferably in a different corner of the at least two corners **(10).** This ensures that the at least one gripping means **(4)** and the at least one outlet **(5)** are on opposite sides of the liquid-tight cavity **(7),** further enhancing the balance of the gravitational center of the bag during irrigation application, i.e., pouring out the irrigation solution.

In a second preferred embodiment of the bag of the present invention according to Figure 3, the bag **(1)** further comprises a top member **(11),** wherein the top member is connected to the connection **(6c)** between the at least two walls **(2)** extending between the at least two corners **(10),** thereby forming a connection **(6e)** between the top member **(11)** and the at least two walls **(2),** wherein the top member **(11)** and the connections **(6e)** between the top member **(11)** and the at least two walls **(2)** form a top part **(12),** and wherein the connections **(6e)** between the top member **(11)** and the at least two walls **(2)** form a top part **(12)** and the connection **(6d)** between the at least two walls **(2)** extending between one of the at least two corners **(10)** form at least two connection joints **(13).**

The advantage if the presence of the top member **(11)** is that the volume of the liquid-tight cavity **(7)** can be increased without increasing the height of the bag or the area occupied by the bag. Furthermore, the top member **(11)** allows for different attachments of the at least one outlet **(5)** and the at least one gripping means **(4)** as described in detailed further below.

For the bag **(1)** of the present invention to facilitate the irrigation solution to leave, it is advantageous if the volume of the liquid-tight cavity **(7)** can be adjusted during operation. Hence, preferably, the top member **(11)** of the bag **(1)** of the present invention is configured to move at least partially into the liquid-tight cavity **(7),** thereby reducing the volume of the liquid-tight cavity **(7).** More preferably, the top member **(11)** is foldable. Most preferably, the top member **(11)** is foldable so that the fold extends into the liquid-tight cavity **(7).** This allows not only a reduction of the volume of the liquid-tight cavity **(7)** by the fold reaching into the liquid-tight cavity **(7),** but also by the at least two walls moving towards each other. Thereby, the volume of the liquid-tight cavity **(7)** can be significantly reduced up to a point of less than 5% of the initial volume, preferably less than 3%, and most preferably less than 1%. The base member could be rigid. In that case a means to facilitate folding has to be present in the base member, i.e., a hinge. Preferably, however, the top member **(11)** comprises a flexible material, preferably a polymer material.

Preferably, in the second preferred embodiment, the at least one outlet **(5)** is located in the top member **(11),** preferably in the connection **(6e)** between the top member **(11)** and the at least two walls **(2).** Most preferably, the at least one outlet **(5)** is located at the connection joint **(13).** This strengthens the above-mentioned effects, as the at least one outlet **(5)** will be held by the two connections **(6d, 6e)** joining in the corners allowing for even higher stability. Furthermore, the at least two walls **(2)** form together with each corner and connections **(6d, 6e)** a funnel shape, further improving the removal effect of the irrigation solution during pouring. In an alternative embodiment of the second preferred embodiment of the present invention, the at least one outlet **(5)** is located in one of the at least two corners **(10).** In an alternative embodiment of the invention, the at least one outlet **(5)** is located in the center of the top member **(11).**

In one embodiment, the at least two corners **(10)** and the at least two connection joints **(13)** are identical. In such an embodiment, the breadth of the top member and the breadth of the at least one walls **(2)** is identical. This allows for maximizing the volume enhancement. However, it also results in removal of the at least two corners **(10),** which otherwise could be used to, e.g., attach the at least one outlet **(5).**

Preferably, in the second preferred embodiment, the at least one gripping means **(4)** is located in the at least two walls **(2)** or in one of the connections **(6d)** between the at least two walls **(2)** extending between one of the at least two corners **(9)** and the connection **(6a)** between the base member **(3)** and the at least two walls **(2),** preferably the at least one gripping means **(4)** is located in one of the connections **(6d)** between the at least two walls **(2)** extending between one of the at least two corners **(9)** and the connection **(6a)** between the base member **(3)** and the at least two walls **(2).** This again achieves that the distance of the at least one gripping means **(4)** to the base member **(3)** is smaller than the distance of the at least one outlet **(5)** to the base member **(3)** resulting in an improved balance of the gravitational center of the bag during irrigation application, i.e., pouring out the irrigation solution.

Hence, most preferably, in the second preferred embodiment of the present invention, the at least one gripping means **(4)** is located in one connection **(6d)** between the at least two walls **(2)** extending between one of the at least two corners **(10)** and the at least one outlet **(5)** is located in the connection **(6e)** between the top member **(11)** and the at least two walls **(2),** preferably at a connection joint **(13),** which is not connected by a connection **(6c)** with the corner **(10)** at which the at least one outlet **(5)** is located. This ensures that the at least one gripping means **(4)** and the at least one outlet **(5)** are on opposite sides of the liquid-tight cavity **(7),** further enhancing the balance of the gravitational center of the bag during irrigation application, i.e., pouring out the irrigation solution.

Preferably, in the present invention, the base member **(3),** the top member **(11),** and/or the at least two walls **(2)** are made from a liquid-tight film, preferably from a liquid-tight film having a thickness of below 300 µm, more preferably of below 250 µm, even more preferably of below 200 µm, and most preferably of below 150 µm. The lower the thickness of the film is, the lower the amount of material used in the bags. Thus, also scrap material eventually produced during production of the bag will be reduced. Furthermore, overall sustainability of the bag is improved. Generally, the thickness of the film is not lower than 60 µm, otherwise, the stability of the standing bag cannot be guaranteed anymore.

Due to the flexible nature of the walls of the bag, said bag can remain partially collapsed not recovering the initial volume of the liquid-tight cavity **(7)** when being put aside between irrigation steps. In contrast, semi-rigid irrigation bottles such as those manufactured by a molding technique (injection stretch blow molding, or extrusion blow molding), which are rigid, regain their original shape when no more pressure is applied by hand on the container, which tends to suck air inside the container. Thus, the bag according to the preferred embodiment of the present invention has the advantage that a lower amount of air is sucked in. Therefore, the risk of airborne contaminant to enter the container and eventually contaminate the irrigation solution is reduced. Additionally, during the pouring out of the liquid, the internal under pressure of the bag, resulting from the pouring out of the liquid is compensated by the collapsing of the walls, which allow to completely eliminate the so called "bubbling effect" seen in rigid and semi rigid containers.

Preferably, the liquid tight film comprises, preferably consists of, a polymer material. Polymeric material offers the best set of properties, i.e., liquid-tightness, flexibility, rigidity, printability, and easy processability. Preferable polymer materials for the present invention are selected from the list consisting of polyethylene, polypropylene, polyethylene terephthalate, polyamide, ethylene vinyl alcohol, or mixtures thereof. Preferably, the polymer material of the present invention is selected from polyethylene or polypropylene.

Preferably, the polymer material of the bag of the present invention has a haze value measured according to ASTM D1003, Procedure B, of less than 60%, preferably less than 30%, more preferably less than 25%, and most preferably less than 15%. This ensures that light is transported through the at least one walls **(2)** or other parts of the bag, allowing the user to look inside the bag and estimate the filling status of the bag and particles contamination. In a preferred embodiment of the bag of the present invention, a scale is printed on at least one of the at least two walls **(2)** allowing for a more accurate determination of the filling state of the bag of the present invention.

Preferably, the polymer material of the bag of the present invention has a glass transition temperature T_{G} measured according to ASTM D3418, of not more than 80 °C, preferably not more than 60 °C, more preferably not more than 40 °C, and most preferably not more than 20 °C. This ensures that the polymer material keeps its flexibility, not only at room temperature, but also at sterilization conditions.

Preferably, the bag **(1)** of the present invention further comprises at least one inlet **(14)** located in the upper part **(9)** or the top part **(12).** Such an inlet facilitates filling the bag before sterilization. More preferably, the at least one inlet **(14)** is selected from the list consisting of a spout, a break-off opening, a twist-off opening, a valve, preferably a non-return valve, an interruption in the connection **(6)** of the at least two walls **(2),** and a tube. The at least one inlet **(14)** is preferably rigid, such as a rigid spout. This ensures operation of the inlet even if the bag is still collapsed to a significant extend. Most preferably, the at least one inlet **(14)** is integrated into the at least two walls **(2)** or the connections **(6).** Thus, preferably, the at least one inlet **(14)** is glued or welded into the at least two walls **(2)** or the connections **(6).**

Preferably, the at least one gripping means **(4)** of the bag of the present invention is selected from the list consisting of a seam area, and a handle. In case the at least one gripping means comprises, preferably consists of, a seam area, the seam width of the seam is more than 10 mm, preferably more than 15 mm, more preferably more than 20 mm, even more preferably more than 25 mm, and most preferably more than 30 mm. Usually, the seam width is not larger than 100 mm. More preferably, the seam area comprises at least one hole. Also more preferably, the seam width of said seam area is not homogenous. Alternatively, the seam thickness of the seam area is not homogenous. Hence, preferably, the surface of the seam area is rough, i.e., a molted surface. The seam area preferably comprises, preferably consists of, the connection **(6b)** of the at least two walls **(2)** or connections derived therefrom such as **(6d),** and **(6e).**

Preferably, the at least one gripping means **(4)** is configured to be used by one hand. Hence, the gripping means **(4)** could be a seam area, which has been broadened to provide an area, which is large enough to be gripped and held by one hand. Alternatively, the width of the seam area could be formed not to be homogenous and/or the seam thickness of the seam area can be not homogenous. Preferably, this area comprises a hole, through which the fingers of the one hand can be pushed through. More preferably, the seam area may be enhanced by a handle, preferably an ergonomic handle. Hence, a handle could be integrated in the seam area, avoiding sharp edges of the seam or seal area. Alternatively, a handle may be attached to the seam area or to the at least two walls **(2).**

Also preferably, the bag of the present invention comprises an overwrap **(15).** Preferably, the overwrap covers a part, more preferably the entire, of each of the at least two walls **(2)** and the base member **(3).** The overwrap has the advantage that the bag is protected from being cut by other objects. Other advantages are protection from dust or external contamination, even gas permeation from external, such as moisture, oxygen, etc.

In a preferred embodiment, the bag of the present invention is at least partially filled with an irrigation solution. Thus, preferably, the bag of the present invention filled with an irrigation solution comprises the irrigation solution and a headspace filled with gas, wherein the gas could be air but also other gases such as inert gases, i.e., nitrogen or noble gases. Preferably, the volume of the headspace is smaller than the volume of the liquid filled part of the liquid-tight cavity **(7).** More preferably, the volume of the headspace is less than 10% of the total volume of the liquid cavity **(7),** preferably less than 5%, more preferably less than 2%, and most preferably less than 1%.

The volume of the headspace can be controlled by either adding further gas through the inlet (increasing the volume) or by removal of the gas (decreasing the volume). The removal of the gas can be preferably carried out by mechanical pressing on the bag or by vacuuming the bag in a position to allow the gas from the headspace to leave the bag during the treatment.

Controlling the volume of the headspace is in particular important for irrigation application. Usually, the bags are sterilized during irrigation application. One typical process of sterilization comprises the step of heating the bag up to at least 105 °C. Thereby, the partial pressure of the gas is increased. If the volume of the headspace is too large, the partial pressure might be able to deform the bag. In a preferred embodiment of the present invention the bag at least partially filled with an irrigation solution has a headspace volume in the range of from 100 to 250 ml.

### Use of the present invention

The present invention further provides a use of the bag according to any of the embodiments described above in an irrigation application. Thereby, the bag is filled with an irrigation solution and is subjected to a sterilization process. During the use, the bag is opened by hand and the irrigation solution is poured on the surgery field or object to be irrigated.

### Process for irrigating an object

The invention further relates to a process for irrigating an object during surgery, providing the following steps:
- providing the object,
- providing a bag according to any of the embodiments described above filled with an irrigation solution being sterilized,
- opening the bag,
- holding the bag with one hand and applying the irrigation solution to the object by pouring.

### Process for producing a bag

Finally, the present invention is concerned with a process for producing a bag according to any of the embodiments described above, the process comprising the steps of:
- providing at least two walls **(2),**
- providing at least one base member **(3),**
- optionally providing at least one gripping means **(4),**
- optionally providing at least one outlet **(5),** and
- connecting the at least two walls **(2)** and the at least one base member **(3)** thereby forming liquid-tight connections **(6),**

wherein the at least two walls **(2),** the at least one base member **(3),** and the liquid-tight connections **(6)** form a liquid-tight cavity **(7),**
wherein the at least one base member **(3)** and the connections **(6a)** between the base member **(3)** and the at least two walls **(2)** form a base part **(8),** and
wherein the at least two walls **(2)** and the connection **(6b)** between the at least two walls **(2)** form an upper part **(9).**

Preferably, the step of providing at least two walls **(2)** comprises the step of cutting out an area from a film. Likewise, preferably, the step of providing the base member **(3)** comprises the step of cutting out an area from a film.

Preferably, the film is a liquid-tight film, preferably a liquid-tight film having a thickness of below 250 µm, more preferably of below 200 µm, and most preferably of below 150 µm. Generally, the thickness of the film is not lower than 60 µm.

Preferably, the liquid-tight film comprises, preferably consists of, a polymer material. Preferable polymer materials for the present invention are selected from the list consisting of polyethylene, polypropylene, polyethylene terephthalate, polyamide, ethylene vinyl alcohol, or mixtures thereof. Preferably, the polymer material of the present invention is selected from polyethylene or polypropylene.

Preferably, the polymer material of the bag of the present invention has a haze value measured according to ASTM D1003, Procedure B, of less than 60%, preferably less than 30%, more preferably less than 25%, and most preferably less than 15%.

Preferably, the polymer material of the present invention has a glass transition temperature T_{G} measured according to ASTM D3418, of not more than 80 °C, preferably not more than 60 °C, more preferably not more than 40 °C, and most preferably not more than 20 °C.

Preferably, the step of connecting comprises a step selected from gluing, melting, or welding, preferably plastic welding.

In order to produce a bag, which is ready for use in irrigation application, an irrigation solution must be filled in the bag. Hence, preferably, after the connecting step, the process further comprises the step of filling the bag with an irrigation solution, preferably via the at least one inlet **(14),** and sealing the bag or closing the inlet. Alternatively, the bag can be filled via the at least one outlet **(5)** and sealed or closed afterwards. After closing the at least one outlet **(5)** and/or at least one inlet **(14),** if present in the bag, and sealing the bag, the bag and the irrigation solution have to be sterilized to be used in an irrigation application. Hence, the process further comprises the step of subjecting the bag and the irrigation solution to a sterilization step.

For the sterilization step it is beneficial that the amount of gas included in the bag is as low as possible. Hence, preferably, the filling and sealing steps are performed while maintaining a vacuum. In an alternative embodiment, the process comprises the step of squeezing the bag before sealing it, to achieve that the gas is pushed out of the bag before sealing.

## Claims

1. A bag suitable for being used in an upright position in an irrigation application, the bag **(1)** comprising
- at least two walls (**2**), and
- at least one base member (**3**)
wherein the at least two walls **(2)** and the at least one base member **(3)** are connected forming liquid-tight connections **(6),**
wherein the at least two walls **(2),** the at least one base member **(3),** and the liquid-tight connections **(6)** form a liquid-tight cavity **(7),**
wherein the at least one base member **(3)** and the connections **(6a)** between the base member **(3)** and the at least two walls **(2)** form a base part **(8),** and
wherein the at least two walls **(2)** and the connection **(6b)** between the at least two walls **(2)** form an upper part **(9).**

2. The bag according to claim 1, wherein the bag further comprises at least one gripping means **(4)** and/or at least one outlet **(5).**

3. The bag according to claims 1 or 2, wherein the base member **(3)** is configured to move at least partially into the liquid-tight cavity **(7)** when the liquid is taken out, thereby reducing the volume of the liquid-tight cavity **(7),** preferably the base member **(3)** is foldable.

4. The bag according to any of the preceding claims, wherein the connection **(6b)** of the at least two walls **(2)** comprises at least two corners **(10).**

5. The bag according to claim 4, wherein the at least one outlet **(5)** is located in the connection **(6c)** between the at least two walls **(2)** extending between the at least two corners **(10),** preferably in one of the at least two corners **(10).**

6. The bag according to any of the preceding claims, wherein the bag **(1)** further comprises a top member **(11),** wherein the top member is connected to the connection **(6c)** between the at least two walls **(2)** extending between the at least two corners **(10),** thereby forming a connection **(6e)** between the top member **(11)** and the at least two walls **(2),**
wherein the top member **(11)** and the connections **(6e)** between the top member **(11)** and the at least two walls **(2)** form a top part **(12),** and
wherein the connections **(6e)** between the top member **(11)** and the at least two walls **(2)** form a top part **(12)** and the connection **(6d)** between the at least two walls **(2)** extending between one of the at least two corners **(10)** form at least two connection joints **(13).**

7. The bag according to claim 6, wherein the at least one outlet **(5)** is located in the top member **(11),** preferably in the connection **(6e)** between the top member **(11)** and the at least two walls **(2),** more preferably at the connection joint **(13).**

8. The bag according to any of the preceding claims, wherein the base member and/or the at least two walls are made from a liquid tight film, preferably from a liquid-tight film having a thickness of below 300 µm, more preferably of below 250 µm, even more preferably of below 200 µm, and most preferably of below 150 µm.

9. The bag according to claim 8, wherein the liquid tight film comprises, preferably consists of, a polymer material, wherein the polymer material is preferably selected from the list consisting of polyethylene, polypropylene, polyethylene terephthalate, polyamide, ethylene vinyl alcohol, or mixture thereof, more preferably is selected from polyethylene or polypropylene.

10. The bag according to claim 9, wherein the polymer material has a haze value measured according to ASTM D1003, Procedure B, of less than 60%, preferably less than 30%, more preferably less than 25%, and most preferably less than 15%.

11. The bag according to any of the preceding claims, wherein the at least one outlet **(4)** comprises a means for opening selected from the list consisting of a tear-off opening, a twist-off opening, a screw-off opening, a break-off opening, a peel-off opening, a flip-flop cap opening, a valve, and combinations thereof.

12. The bag according to any of the preceding claims, wherein the at least one gripping means **(4)** is selected from a seam area or a handle.

13. The bag according to claim 12, wherein the seam area comprises at least one hole or the width of the seam area is not homogenous.

14. The use of the bag according to any of the preceding claims 1 to 13 in an irrigation application.

15. Process for producing a bag according to any of claim 1 to 13, the process comprising the steps of:
- providing at least two walls (**2**),
- providing at least one base member (**3**),
- optionally providing at least one gripping means (**4**),
- optionally providing at least one outlet (**5**), and
- connecting the at least two walls (**2**) and the at least one base member (**3**) thereby forming liquid-tight connections (**6**),
wherein the at least two walls **(2),** the at least one base member **(3),** and the liquid-tight connections **(6)** form a liquid-tight cavity **(7),**
wherein the at least one base member **(3)** and the connections **(6a)** between the base member **(3)** and the at least two walls **(2)** form a base part **(8),** and
wherein the at least two walls **(2)** and the connection **(6b)** between the at least two walls **(2)** form an upper part **(9).**
